# FASCICULE DE BREVET EUROPEEN

(11) **EP 1 685 853 B1**
(45) Date de publication et mention de la délivrance du brevet: **06.05.2009**
(21) Numéro de dépôt: 06100965.0
(22) Date de dépôt: 27.01.2006
(51) Int. Cl.: A61L 2/08

(54) **Installation et procede pour la sterilisation d'objets par bombardement d'electrons a faible energie**
Vorrichtung und Verfahren zur Sterilisation von Gegenständen durch Beschuss mit Elektronen niedriger Energie
Installation and process for the sterilisation of objects by bombardment with low energy electrons

(30) Priorité: 01.02.2005 FR 0550287
(43) Date de publication de la demande: 02.08.2006
(73) Titulaire: Linac Technologies SAS, 91400 Orsay (FR)
(72) Inventeur: Foncuberta Philippe, 41100, Vendome (FR); Morisseau, Didier, 92160, Antony (FR); Coupez, Arnaud, 91530 Saint Cheron (FR)
(74) Mandataire: Poulin, Gérard

(56) Documents cités:
- WO-A-99/39750
- US-A- 4 599 216
- US-A1- 2003 091 468
- US-A1- 2004 245 481

## Description

### DOMAINE TECHNIQUE

La présente invention se rapporte de façon générale au domaine de la stérilisation d'objets par bombardement d'électrons à faible énergie sur la surface extérieure de ces objets.

Elle s'applique de façon particulière mais non exclusive à la stérilisation par bombardement d'électrons à faible énergie d'objets de forme sensiblement parallélépipédique rectangle, tels que des objets dénommés « tubs » correspondant à des récipients fermés dans lesquels reposent une multitude d'éléments de préférence pré-stérilisés par voie chimique, comme par exemple des seringues médicales.

Plus particulièrement, l'invention concerne une installation pour la stérilisation d'objets par bombardement d'électrons à faible énergie sur la surface de ces objets, ainsi qu'un procédé de stérilisation apte à être mis en oeuvre par une telle installation.

### ÉTAT DE LA TECHNIQUE ANTÉRIEURE

De l'art antérieur, on connaît des installations permettant d'assurer la stérilisation d'objets de forme sensiblement parallélépipédique rectangle par bombardement d'électrons à faible puissance, c'est-à-dire de puissance inférieure à environ 400 KeV.

Pour ce faire, ces installations comportent typiquement trois sources de faible énergie du type accélérateurs/canons d'environ 200 KeV, et sont disposées à 120° les unes par rapport aux autres autour d'une chambre de traitement à travers laquelle les objets sont mis en translation afin d'y être traités. De cette façon, lors de son passage en continue dans la chambre de traitement, chaque objet voit sa surface extérieure illuminée simultanément sur 360° par la combinaison des trois faisceaux d'électrons issus respectivement des sources précitées, qui ont été judicieusement positionnées.

Ce type d'installation s'est révélé satisfaisant, notamment en raison de son efficacité liée à la légère pénétration du faisceau d'électrons et à l'effet sporicide rencontrés, et également en raison de sa rapidité de traitement ainsi que de sa sécurité procurée. Le document WO 99/39750 ainsi que le document US 2003/091468 divulguent une telle installation.

Néanmoins, cette installation présente un inconvénient majeur résidant dans le fait qu'elle nécessite trois sources distinctes de faible énergie du type accélérateurs/canons pour assurer la stérilisation par irradiation de l'intégralité de la surface extérieure de chaque objet. Bien évidemment, cette contrainte liée à la présence obligatoire de trois sources complexes de faible énergie rend l'installation extrêmement coûteuse, et donc pas entièrement optimisée.

Par ailleurs, il est noté que l'encombrement particulièrement important de cette installation constitue également un autre inconvénient.

### EXPOSÉ DE L'INVENTION

L'invention a donc pour but de proposer une installation pour la stérilisation d'objets par bombardement d'électrons à faible énergie sur la surface de ces objets, ainsi qu'un procédé de stérilisation apte à être mis en oeuvre par une telle installation, cette installation et ce procédé remédiant au moins partiellement aux inconvénients mentionnés ci-dessus relatifs aux réalisations de l'art antérieur.

Pour ce faire, l'invention a tout d'abord pour objet une installation selon la revendication 1.

En d'autres termes, l'une des particularités de l'invention consiste à établir un mouvement relatif de rotation entre les moyens de stérilisation et l'objet, de manière à ce que la surface extérieure de ce dernier soit traitée en continue et progressivement durant ce mouvement relatif de rotation.

Dans l'installation selon l'invention, il n'est avantageusement plus nécessaire de faire appel à trois sources distinctes de faible énergie destinées à illuminer conjointement et simultanément la surface extérieure d'un objet sur 360°. En effet, avec l'agencement proposé, l'objet à stériliser peut être traité à l'aide d'une unique source de faible énergie dont le faisceau d'électrons selon l'axe A, à un instant t quelconque du mouvement relatif de rotation, n'illumine qu'un secteur angulaire inférieur à 360° de la surface extérieure de l'objet. C'est alors l'application du mouvement relatif de rotation opéré selon l'axe A', traversant en permanence la surface extérieure de l'objet et étant de préférence orthogonal à l'axe A, qui permet une illumination et un traitement progressif de cette surface extérieure sur 360°.

Bien évidemment, le fait de ne nécessiter qu'une unique source de faible énergie du type accélérateurs/canons engendre une réduction significative du coût global ainsi qu'un encombrement réduit par rapport à ceux associés aux installations connues de l'art antérieur.

L'installation selon l'invention est préférentiellement réalisée de manière à ce que chaque objet à stériliser soit pris en charge de façon automatique durant l'intégralité de son passage entre un convoyeur situé à l'entrée de l'installation, et un isolateur de production situé à la sortie de cette installation.

A ce titre, il est noté que l'installation est adaptée pour recevoir une pluralité d'objets défilant en continue sur le convoyeur d'entrée, chacun d'entre eux étant alors destiné à pénétrer successivement à l'intérieur de la chambre de traitement. L'installation est de préférence destinée à assurer des cadences dite « faibles » de l'ordre de 1 objet/minute, correspondant par exemple à 30 secondes de transfert entre l'entrée et la sortie de l'installation, et à 30 secondes effectives de traitement par bombardement d'électrons en vue de la stérilisation de la surface extérieure de l'objet.

L'installation selon l'invention, pour laquelle le faisceau d'électrons généré par les moyens de stérilisation illumine l'objet en permanence durant le mouvement relatif de rotation selon l'axe A', est naturellement adaptée pour le traitement d'objet de toute forme, dont celle préférée d'un « tub », par exemple pour seringues médicales, prenant sensiblement la forme d'un parallélépipède rectangle. Bien entendu, d'autres formes d'objet à stériliser en surface peuvent être envisagées, telles que la forme cylindrique de section circulaire, sans sortir du cadre de l'invention.

Par ailleurs, il est noté que l'installation est naturellement conçue de façon à assurer le respect de la régulation du flux d'air à travers la chambre de traitement pendant les phases de transfert de l'objet au sein de l'installation.

D'autre part, en opérant une telle rotation selon l'axe A'' précité, il devient alors possible, après avoir balyé la surface sur 360° à l'aide du premier module mécanique et autour de l'axe A', d'envisager de traiter les faces de l'objet non-traitées durant la première rotation selon l'axe A'. Cette spécificité est particulièrement intéressante lorsque l'installation est destinée à la stérilisation d'objets de forme sensiblement parallélépipèdique rectangle, tels que des tubs.

De préférence, le premier module mécanique est piloté pour coopérer, durant la stérilisation de l'objet à traiter situé à l'intérieur de ladite chambre de traitement, avec l'un quelconque des deux éléments pris parmi l'objet et les moyens de stérilisation, de manière à mettre cet élément en rotation selon l'axe A'. De cette manière, il est à comprendre que si le mouvement relatif de rotation précité est préférentiellement obtenu en appliquant une rotation à l'objet tout en maintenant les moyens de stérilisation fixes sur l'installation, le cas inverse peut bien évidemment être envisagé.

Comme cela a été évoqué ci-dessus, l'installation est préférentiellement conçue de sorte que l'objet à traiter constitue l'élément destiné à coopérer avec le premier module mécanique afin d'être mis en rotation selon l'axe A', les moyens de stérilisation étant alors montées fixement sur l'installation.

Dans un tel cas, on peut prévoir que le premier module mécanique est pourvu de moyens de préhension de l'objet à traiter, ce premier module comportant des premiers moyens de mise en mouvement destinés à amener l'objet, coopérant avec les moyens de préhension, dans la chambre de traitement de façon à ce qu'une première face de cet objet soit traversée par l'axe A, de préférence perpendiculairement. De plus, le premier module mécanique comprend également des seconds moyens de mise en mouvement conçus de manière à mettre en rotation l'objet et les moyens de préhension selon l'axe A' orienté orthogonalement par rapport à l'axe A et par rapport à une seconde face de l'objet, le second module mécanique comprenant quant à lui des troisièmes moyens de mise en mouvement conçus de manière à mettre en rotation d'environ 90°, à l'intérieur de la chambre de traitement, l'objet libéré des moyens de préhension, selon l'axe A'' orienté orthogonalement par rapport à l'axe A' et par rapport à l'une quelconque des faces de l'objet perpendiculaires à la seconde face mentionnée ci-dessus.

Avec une telle configuration disposant des premier et second modules mécaniques qui viennent d'être décrits, l'installation est alors tout à fait adaptée pour stériliser les six faces d'un objet parallélépipédique rectangle, comme cela sera détaillée ci-après.

De préférence, le second module mécanique comporte des quatrièmes moyens de mise en mouvement conçus de manière à mettre l'objet en translation selon l'axe A", afin d'écarter celui-ci desdits moyens de préhension lors de sa rotation d'environ 90° selon ce même axe A" engendrée par les troisièmes moyens de mise en mouvement. Bien entendu, ces quatrièmes moyens permettent également de ramener l'objet vers les moyens de préhension, après que cette rotation ait été effectuée. A cet égard, il est noté que le premier module mécanique est conçu et piloté de manière à ce que ses moyens de préhension réengagent l'objet de forme sensiblement parallélépipédique rectangle ayant subi la rotation d'environ 90° générée par les troisièmes moyens de mise en mouvement, afin que cet objet puisse être mis en rotation selon l'axe A' par les seconds moyens de mise en mouvement. Naturellement, cette seconde rotation selon l'axe A' a essentiellement pour but de stériliser les deux faces parallèles de l'objet parallélépipédique qui n'ont pas été traitées lors de la première rotation de 360° selon ce même axe. Néanmoins, elle peut éventuellement être utilisée pour compléter le traitement des deux autres faces déjà stérilisées lors de la première rotation, mais également illuminées par le faisceau d'électrons durant la seconde rotation de l'objet selon cet axe A'.

Toujours de façon préférentielle, l'axe A'' est orienté selon une direction de la hauteur Z de l'installation, et l'axe A est orienté selon une direction d'avancement des objets X au sein de l'installation, les directions X et Z étant orthogonales entre elles. A cet égard, l'axe A est également de préférence orienté selon la direction de la hauteur Z.

L'installation comprend en outre un sas d'entrée et un sas de sortie entre lesquels se situe la chambre de traitement communiquant avec celles-ci, cette chambre de traitement ainsi que les deux sas d'entrée et de sortie étant alignés selon la direction X. Dans un tel cas, on prévoit alors que les premiers moyens de mise en mouvement sont conçus de manière à permettre un déplacement en translation selon la direction X de l'objet maintenu par les moyens de préhension, d'une part entre le sas d'entrée et la chambre de traitement, et d'autre part entre cette dernière et le sas de sortie.

De préférence, les deux sas d'entrée et de sortie en position fermée et la chambre de traitement forment conjointement une enceinte blindée non étanche, qui permet donc une protection biologique totale vis-à-vis du faisceau d'électrons, mais qui est tout de même susceptible de laisser passer l'air, essentiellement au niveau des fermetures des deux sas.

Chacun des deux sas d'entrée et de sortie est de préférence associé à un élévateur orienté selon la direction Z, ce qui permet de réduire considérablement l'encombrement d'une telle installation. En outre, chacun des deux élévateurs comporte un plateau mobile blindé servant de support pour l'objet, ce plateau étant conçu pour assurer la fermeture du sas auquel il est associé, et constituant donc une partie de l'enceinte blindée non étanche mentionnée précédemment.

D'autre part, les deux élévateurs coopèrent respectivement avec un convoyeur d'entrée et un convoyeur de sortie, chacun apte à assurer le déplacement des objets. A ce titre, le convoyeur de sortie débouche dans un isolateur de production dans lequel sont acheminés les objets stérilisés.

D'autre part, l'invention a également pour objet un procédé de stérilisation d'objets par bombardement d'électrons à faible énergie sur la surface extérieure de ces objets, ce procédé possédant la particularité d'être mis en oeuvre à l'aide d'une installation telle que celle qui vient d'être décrite ci-dessus.

En d'autres termes, ce procédé consiste globalement, après avoir amené un objet à traiter à l'intérieur de la chambre de traitement, à appliquer à l'aide du premier module mécanique un mouvement relatif de rotation entre l'objet et les moyens de stérilisation, selon un axe A' choisi de sorte que l'axe A du faisceau d'électrons généré par les moyens de stérilisation traverse la surface extérieure de l'objet en permanence durant ce mouvement relatif de rotation selon cet axe A'.

D'autres avantages et caractéristiques de l'invention apparaîtront dans la description détaillée non limitative ci-dessous.

### BRÈVE DESCRIPTION DES DESSINS

Cette description sera faite au regard des dessins annexés parmi lesquels ;
- la figure 1 représente une vue en perspective d'une installation pour la stérilisation d'objets par bombardement d'électrons à faible énergie, selon un mode de réalisation préféré de la présente invention ;
- la figure 2 représente une vue similaire à celle de la figure 1, dans une version où certains éléments de l'installation ont été volontairement omis, pour des raisons de clarté ;
- la figure 3 représente une vue en perspective d'une partie du premier module mécanique de l'installation montrée sur la figure 1 ;
- la figure 4 représente une vue partielle et agrandie en perspective de la figure 3, montrant plus spécifiquement les moyens de préhension du premier module mécanique ;
- la figure 5 représente une vue en perspective du second module mécanique de l'installation montrée sur la figure 1 ;
- les figures 6a et 6b représentent des vues de face schématisant la position de l'objet à différents stades de son traitement dans l'installation ; et
- la figure 7 représente une vue partielle en perspective d'une installation pour la stérilisation d'objets par bombardement d'électrons à faible énergie, selon un autre mode de réalisation préféré de la présente invention.

### EXPOSÉ DÉTAILLÉ DE MODES DE RÉALISATION PRÉFÉRÉS

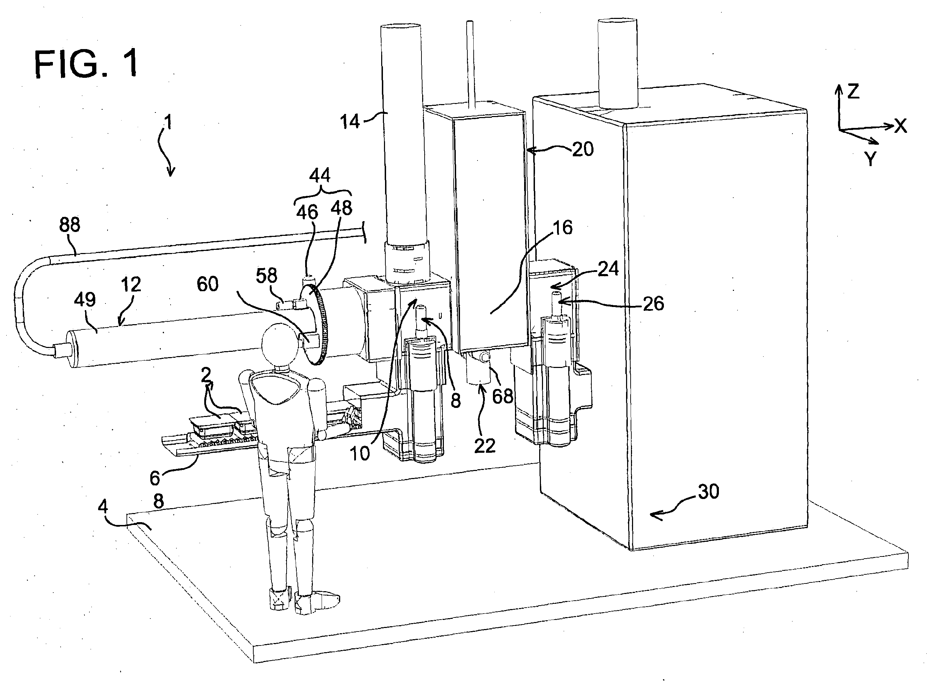

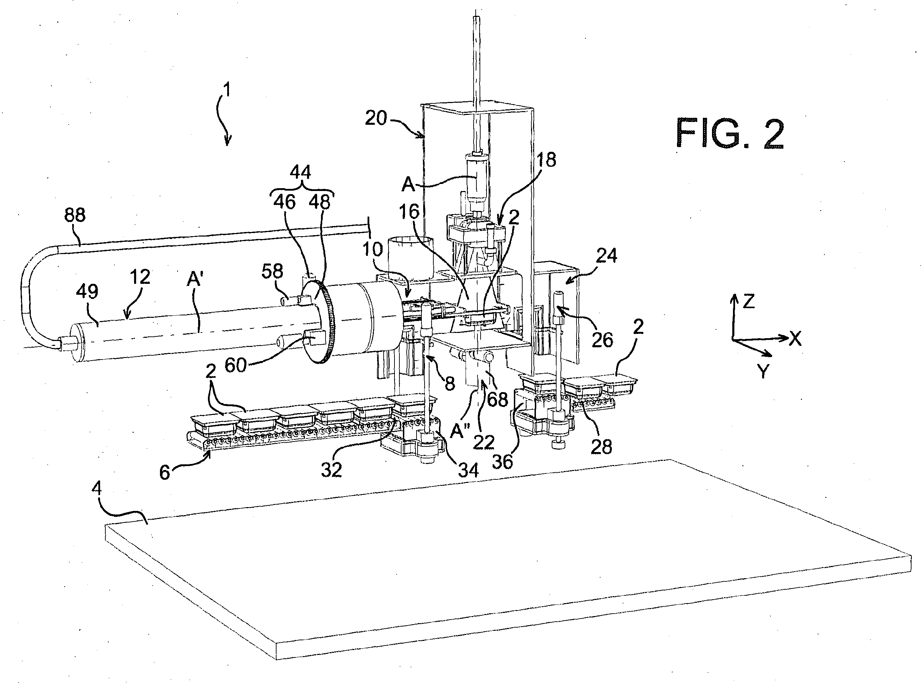

En référence tout d'abord conjointement aux figures 1 et 2, on peut apercevoir une installation 1 pour la stérilisation d'objets 2 par bombardement d'électrons à faible énergie, cette installation 1 étant particulièrement destinée au traitement d'objets de forme sensiblement parallélépipédique rectangle.

Comme cela a été évoqué précédemment, cette forme correspond notamment à celle de tubs dans lesquels reposent une multitude d'éléments ayant été pré-stérilisés par voie chimique, tels que des seringues médicales pouvant par exemple être au nombre de cent par tub.

L'installation 1 est posée sur un sol 4, que l'on peut assimiler à un plan horizontal. A ce titre, on peut noter que la description sera faite en référence à une direction X parallèle au sol 4 et correspondant à une direction d'avancement des objets au sein de l'installation, une direction Y correspondant à une direction transverse de l'installation également parallèle au sol 4, ainsi qu'à une direction Z correspondant à une direction de la hauteur qui est quant à elle orthogonale à ce même sol 4, les directions X, Y et Z étant orthogonales entre elles.

Globalement, l'installation comporte les éléments suivants : un convoyeur d'entrée 6, un élévateur d'entrée 8, un sas d'entrée 10, un premier module mécanique 12 de mise en mouvement des objets 2, une cheminée d'extraction d'ozone 14, une chambre de traitement 16 également dénommée « tunnel de tir », des moyens de stérilisation 18 situés à l'intérieur d'un coffrage 20, un second module mécanique 22 de mise en mouvement des objets 2, un sas de sortie 24, un élévateur de sortie 26, un convoyeur de sortie 28, et un isolateur de production 30 dans lequel sont acheminés les objets stérilisés 2.

Le convoyeur d'entrée 6 est un convoyeur gravitaire à rouleaux assurant un déplacement des objets 2 à traiter, sensiblement selon la direction X. Il est équipé d'une écluse 32 en partie basse, pilotée par un électro-aimant (non représenté) ou par un autre type d'actionneur connu de l'homme du métier, et permettant de libérer un à un les objets 2 afin que ceux-ci pénètrent automatiquement par gravité et successivement dans l'élévateur d'entrée 8. Comme on peut le voir sur la figure 1, la portion finale du convoyeur d'entrée 6 est entourée d'une paroi protectrice, de préférence réalisée en acier inoxydable, cette paroi s'étendant jusque dans une partie basse de l'élévateur d'entrée 8.

L'élévateur d'entrée 8 est également pourvu d'une double-paroi protectrice dans sa partie haute, qui est composée d'un revêtement étanche du type en acier inoxydable, ainsi que d'un revêtement blindé de préférence réalisé en plomb. Au même titre que pour les autres éléments de blindage de l'installation 1, le revêtement blindé entourant l'élévateur 8 sert essentiellement à assurer une protection biologique totale vis-à-vis du faisceau d'électrons émis par les moyens de stérilisation 18 durant le traitement.

En outre, l'élévateur 8 présente un plateau mobile blindé 34 servant de support pour un objet 2 issu du convoyeur 6, ce plateau 34 étant susceptible d'être mis en mouvement selon la direction Z. Il est noté qu'en position basse, le plateau 34 se situe sensiblement dans la continuité du convoyeur d'entrée 6, dans la direction X.

Au droit de l'élévateur 8 dans la direction Z, c'est-à-dire vers le haut, se situe le sas d'entrée 10 délimité par une double-paroi protectrice en forme de boîte parallélépipédique ouverte vers la chambre de traitement 16, située de façon adjacente dans la direction X. Cette double-paroi protectrice est aussi composée d'un revêtement étanche du type en acier inoxydable, ainsi que d'un revêtement blindé de préférence réalisé en plomb, présentant une épaisseur de l'ordre de 15 mm.

Le premier module mécanique 12 est monté fixement sur une face YZ extérieure de cette boîte, c'est-à-dire orientée dans un plan défini par les directions Y et Z, tandis que la cheminée d'extraction d'ozone 14 est montée sur une face XY supérieure de cette même boîte, c'est-à-dire orientée dans un plan défini par les directions X et Z. A cet égard, il est précisé que le plateau blindé 34, en position haute, est destiné à constituer une face XY inférieure de la boîte de protection mentionnée ci-dessus, dont une face YZ intérieure a volontairement été omise pour assurer la communication entre le sas d'entrée 10 et la chambre de traitement 16 adjacente. Ainsi, le plateau 34, en fonction de son positionnement, est en mesure d'assurer l'ouverture et la fermeture du sas d'entrée 10 équipé de la protection biologique qui vient d'être présentée.

La chambre de traitement 16 est située dans le prolongement du sas d'entrée 10 selon la direction X, et est également délimitée par une double-paroi protectrice sensiblement en forme de boîte parallélépipédique ouverte de chaque côté dans cette même direction X. Ici encore, la double-paroi protectrice est composée d'un revêtement étanche du type en acier inoxydable, ainsi que d'un revêtement blindé de préférence réalisé en plomb, présentant une épaisseur de l'ordre de 15 mm. Il est néanmoins noté que la face XY supérieure de cette boîte, c'est-à-dire orientée dans un plan défini par les directions X et Y, ne présente qu'un revêtement étanche sur lequel sont agencés les moyens de stérilisation 18. Ces derniers sont d'ailleurs situés à l'intérieur du coffrage blindé 20 réalisé en plomb sur une épaisseur d'environ 15 mm, et étant agencé au-dessus de la boîte précitée délimitant la chambre de traitement 16.

Les moyens de stérilisation 18 aptes à générer un faisceau d'électrons à faible énergie selon un axe A prennent de préférence la forme d'un canon ou accélérateur à électrons de puissance inférieure à 400 KeV, et par exemple de l'ordre de 200 KeV. Ce canon 18 est donc monté fixement sur l'installation 1 à la périphérie de la chambre de traitement 16, et de préférence de façon adjacente dans la direction Z comme cela est clairement visible sur la figure 2. Ainsi, ils sont capables d'émettre un faisceau d'électrons selon un axe A traversant la chambre de traitement 6 et étant orienté parallèlement à la direction Z. Par ailleurs, ces moyens de stérilisation 18 peuvent, de façon connue, comprendre en bout de canon un cornet de balayage (non référencé) du faisceau d'électrons, ce cornet délimitant une fenêtre de sortie pour les électrons et étant préférentiellement orienté selon la direction X.

Le second module mécanique 22, qui sera détaillé ultérieurement, est monté sur une face XY inférieure de la boîte précitée.

Le sas de sortie 24 est situé dans le prolongement de la chambre de traitement 16 selon la direction X, et est aussi délimité par une double-paroi protectrice en forme de boîte parallélépipédique ouverte vers la chambre de traitement 16. Cette double-paroi protectrice est composée d'un revêtement étanche du type en acier inoxydable, ainsi que d'un revêtement blindé de préférence réalisé en plomb, présentant une épaisseur de l'ordre de 15 mm.

Au droit du sas de sortie 24 dans une direction opposée à la direction Z, c'est-à-dire vers le bas, se situe l'élévateur de sortie 26 qui est également pourvu d'une double-paroi protectrice dans sa partie haute, composée d'un revêtement étanche du type en acier inoxydable, ainsi que d'un revêtement blindé de préférence réalisé en plomb.

En outre, l'élévateur 26 présente un plateau mobile blindé 36 servant de support pour un objet 2 issu du sas de sortie 24, ce plateau 36 étant susceptible d'être mis en mouvement selon la direction Z. A cet égard, il est précisé que le plateau blindé 36, en position haute, est destiné à constituer une face XY inférieure de la boîte de protection délimitant le sas de sortie 24, dont une face YZ intérieure a volontairement été omise pour assurer la communication entre ce sas d'entrée 24 et la chambre de traitement 16 adjacente. Ainsi, le plateau 36, en fonction de son positionnement, est en mesure d'assurer l'ouverture et la fermeture du sas de sortie 24.

De plus, il est noté qu'en position basse, le plateau 36 se situe sensiblement dans la continuité du convoyeur de sortie 28, dans la direction X. Tout comme le convoyeur d'entrée 6, le convoyeur de sortie 28 est un convoyeur gravitaire à rouleaux assurant un déplacement des objets 2 à traiter, sensiblement selon la direction X. Comme on peut le voir sur la figure 1, la portion initiale du convoyeur de sortie est entourée d'une paroi protectrice, de préférence réalisée en acier inoxydable, cette paroi s'étendant jusque dans une partie basse de l'élévateur de sortie 26.

Enfin, il est noté que le convoyeur de sortie 28 débouche à l'intérieur de l'isolateur de production 30.

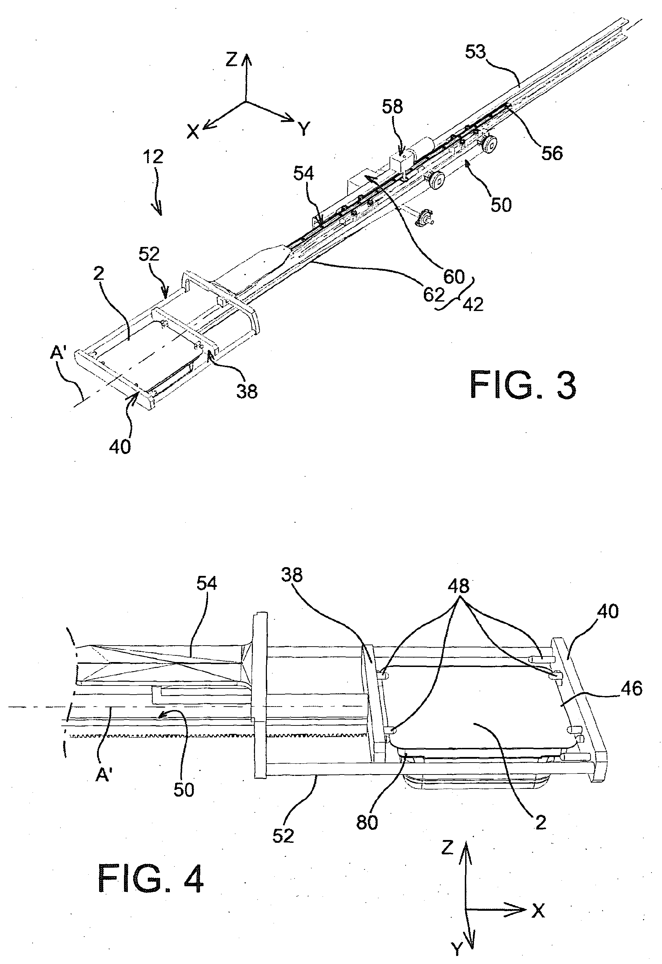

En référence à présent aux figures 3 et 4, il va être décrit le premier module mécanique 12.

Globalement, ce premier module mécanique 12 comporte des moyens de préhension 38, 40 de l'objet 2 à traiter, des premiers moyens de mise en mouvement 42 destinés à mettre ledit objet 2 en translation à l'intérieur de l'installation 1 selon un axe A' orienté selon la direction X et donc orthogonal à l'axe A, ainsi que des seconds moyens de mise en mouvement destinés à mettre ledit objet 2 en rotation à l'intérieur de l'installation 1, selon ce même axe A'. Il est noté que ces seconds moyens sont uniquement représentés sur les figures 1 et 2 sur lesquelles ils portent la référence 44. En effet, ces seconds moyens 44 ont pour fonction de faire tourner tout l'ensemble représenté sur la figure 3, par l'intermédiaire d'un pignon motorisé 46 et d'un engrenage 48 d'axe confondu avec l'axe A', montés extérieurement par rapport à l'enceinte blindée formée conjointement par les sas 10, 26 et la chambre de traitement 16. En outre, toujours en référence à la figure 1, le premier module 12 comporte une double-paroi protectrice étanche et blindée 49 en forme d'enveloppe de section circulaire, dans laquelle est inséré l'ensemble montré sur cette figure 3.

Les moyens de préhension présentent deux surfaces de préhension parallèles 38 et 40, orientées sensiblement selon des plans YZ, et chacune pourvue de picots 49 s'étendant selon la direction X pour engager un rebord circonférentiel 47 de l'objet 2, comme on peut l'apercevoir sur la figure 4. Etant donné que les moyens de préhension sont destiné à maintenir l'objet 2 à traiter durant la rotation de ce dernier selon l'axe A', ces picots 49 sont bien entendu répartis de manière à se situer au-dessus et en dessous du bord circonférentiel 46 précité.

La surface 38 est agencée à l'extrémité d'un bras principal 50 du premier module mécanique 12, ce bras 50 s'étendant selon la direction X étant destiné à coulisser dans cette même direction par rapport à deux rails parallèles 53 dont un seul a été représenté sur la figure 3, pour des raisons évidentes de clarté. Par ailleurs, la surface 40, la plus extérieure, appartient à un cadre 52 agencé à l'extrémité d'un bras secondaire 54 du premier module mécanique 12, ce cadre 52 étant de dimension suffisamment important pour pouvoir être traversé par un objet 2 dans la direction Z. De plus, le bras 54 s'étend également selon la direction X et est destiné à coulisser selon la direction X par rapport au bras principal 50 au-dessus duquel il est préférentiellement agencé. Cette faculté est offerte par la présence d'une crémaillère 56 sur le bras secondaire 54, qui est pilotée par un pignon motorisé 58. Ainsi, en actionnant ce pignon motorisé 58, il est donc possible de régler la distance selon la direction X entre les deux surfaces de préhension 38, 40, principalement de manière à adapter cette même distance par rapport aux dimensions de l'objet 2 à maintenir.

Toujours en référence à cette figure 3, on peut voir que les premiers moyens de mise en mouvement 42 destinés à assurer le coulissement du bras principal 50 sur lequel est embarqué le bras secondaire 54, comprennent un pignon motorisé 60 et une crémaillère 62 agencée sur ce même bras 50.

De la même façon que pour le pignon motorisé 46 du premier module mécanique 12, les moteurs des pignons motorisés 58 et 60 de ce module sont agencés extérieurement au blindage de l'installation, comme on peut le voir sur les figures 1 et 2.

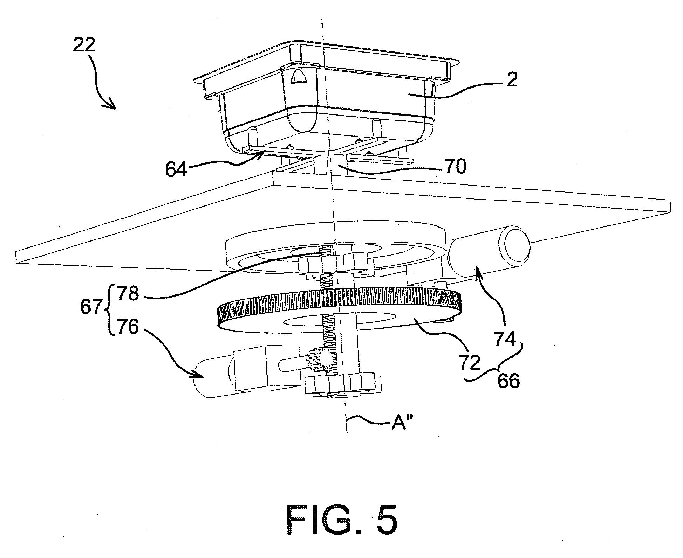

En référence à la figure 5, il va être décrit le second module mécanique 22.

Globalement, ce second module mécanique 22 comporte des moyens de support 64 de l'objet 2 à traiter, des troisièmes moyens de mise en mouvement 66 destinés à mettre l'objet 2 en rotation à l'intérieur de l'installation 1 selon un axe A'' orienté selon la direction Z, et de préférence confondu avec l'axe A du faisceau d'électrons, et des quatrièmes moyens de mise en mouvement 67 destinés à mettre l'objet 2 en translation à l'intérieur de l'installation 1 selon cet axe A''. En outre, on peut voir sur la figure 1 que le second module 22 comporte une double-paroi protectrice étanche et blindée 68 en forme d'enveloppe de section circulaire, dans laquelle est inséré l'ensemble montré sur cette figure 5.

Le module 22 présente un bras 70 orienté selon l'axe A", et à l'extrémité supérieure duquel se trouvent les moyens de support 64 sur lesquels l'objet 2 est capable de reposer par gravité. En outre, ce bras 70 est couplé aux troisièmes moyens de mise en mouvement 66 comprenant un engrenage 72 lié mécaniquement à ce bras 70, ce dernier étant d'axe confondu avec l'axe A'' et coopérant avec un pignon motorisé 74 appartenant également aux troisièmes moyens 66. Les éléments 72, 74 sont préférentiellement agencés extérieurement au blindage de l'installation, et plus spécifiquement à celui procuré par la double-paroi protectrice 68.

D'autre part, les quatrièmes moyens de mise en mouvement 67 comprennent quant à eux un pignon motorisé 76 dont le moteur est également prévu extérieurement au blindage procuré par la double-paroi protectrice 68, ce pignon motorisé 76 coopérant avec une crémaillère directement pratiquée sur le bras 70, en vue d'assurer un coulissement de celui-ci dans la direction Z.

Le fonctionnement de l'installation 1 rencontré lors de la mise en oeuvre d'un procédé de stérilisation d'objets par bombardement d'électrons à faible énergie, selon un mode de réalisation préféré de la présente invention, va à présent être décrit.

Tout d'abord, il est noté que l'ensemble des opérations présentées ci-dessous peuvent être automatisées à l'aide de moyens informatiques appropriés (non représentés), de sorte qu'un opérateur a uniquement besoin de placer manuellement les objets 2 à stériliser sur le convoyeur d'entrée 6, avant que chacun d'entre eux soit entièrement pris en charge de façon automatique durant l'intégralité de son passage entre ce même convoyeur 6, et l'isolateur de production 30.

L'actionnement de l'écluse 32 a pour conséquence de libérer un objet 2 du convoyeur d'entrée 6, qui peut alors automatiquement pénétrer par gravité dans l'élévateur d'entrée 8, et plus particulièrement glisser sur le plateau 34 maintenu en position basse.

Ensuite, le plateau 34 est élevé jusque dans une position haute dans laquelle il ferme le sas d'entrée 10, et dans laquelle l'objet 2 supporté par ce plateau 34 a pénétré entre les deux surfaces 38, 40 des moyens de préhension, dont l'écartement avait été fixé en conséquence. Les pignons motorisés 58 et 60 sont actionnés de manière à ce que les surfaces 38, 40 se rapprochent l'une de l'autre, et que leurs picots 49 engagent respectivement le rebord circonférentiel 47 de deux faces de l'objet 2 opposées et orientées dans des plans parallèles YZ, parmi lesquelles on note une face dite seconde face indiquée par la référence 80 sur la figure 4.

Cette seconde face 80 est donc disposée orthogonalement par rapport à l'axe A', et est de préférence traversée par ce dernier.

Ensuite, le plateau 34 est légèrement redescendu tout en assurant la continuité de la protection biologique de plomb, et il est procédé à l'actionnement des premiers moyens de mise en mouvement 42 afin de déplacer l'objet 2 en translation selon la direction X, vers la chambre de traitement 16, par déplacement du bras principal 50 dans cette même direction. Le pignon motorisé 60, générant ce déplacement pendant lequel le pignon motorisé 58 reste bien évidemment inactif, s'arrête lorsqu'une face supérieure de l'objet 2 orientée selon un plan XY est traversée orthogonalement par l'axe A', et de préférence en son milieu. Cette face supérieure, dite première face, est indiquée par la référence 82 sur la figure 4.

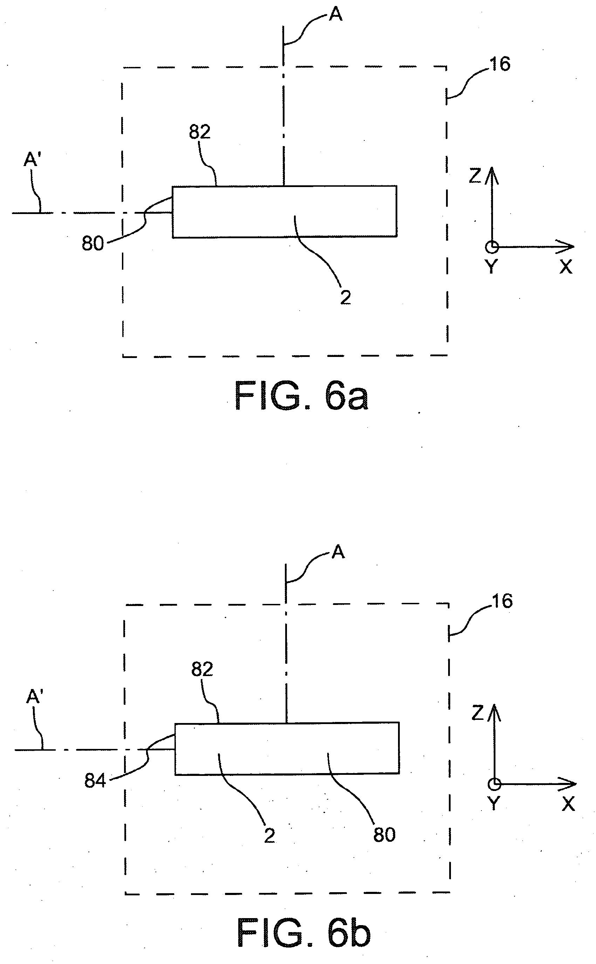

A cet instant, l'objet 2 à traiter par bombardement d'électrons en surface se trouve à l'intérieur de la chambre de traitement 16, comme cela est représenté schématiquement sur la figure 6a. Le plateau 36 de l'élévateur 26 se trouve en position haute de manière à fermer de manière non étanche le sas de sortie 24, de sorte que ce dernier constitue conjointement avec la sas d'entrée 10 et la chambre 16 une enceinte blindée non étanche, conférant une protection biologique totale vis-à-vis du faisceau d'électrons. La fermeture non étanche du sas d'entrée 10 et du sas de sortie 24 permet un apport d'air dans l'enceinte précitée, qui est utile à la dilution de l'ozone produite par le bombardement d'électrons et destinée à s'échapper par la cheminée 14. D'autre part, cette fermeture étanche donne un sens de circulation d'air favorable à la protection de l'asepsie de l'isolateur 30.

A ce stade, les moyens de stérilisation 18 sont alors mis en oeuvre de manière à générer le faisceau d'électrons mentionné ci-dessus selon l'axe A, d'une façon telle que la tache focale de ce faisceau soit fixe et puisse illuminer sensiblement toute la superficie de la première face 82, ou bien encore de sorte qu'un balayage du faisceau de tache focale plus faible soit pratiqué selon la direction X. En effet, dans ce dernier cas, la fréquence d'oscillation retenue est suffisamment importante pour que le faisceau illumine toute la surface de l'objet à traiter à la vitesse de rotation pratiquée.

Simultanément, les seconds moyens de mise en mouvement 44 du premier module mécanique 12 sont actionnés par l'intermédiaire du pignon motorisé 46, afin de mettre l'objet 2 en rotation selon l'axe A'.

Cette phase de rotation de l'objet 2 selon l'axe A', durant laquelle l'axe A du faisceau d'électrons fixe traverse en permanence l'une des faces de l'objet 2, est opérée jusqu'à ce que cet objet ait effectué un tour complet, à savoir jusqu'à ce que l'ensemble du premier module mécanique 12 montré sur la figure 3 ait effectué une rotation de 360° autour de cet axe A'.

Ainsi, durant cette première phase de rotation de l'objet 2, ceux sont quatre des six faces de celui-ci qui ont pu être traitées par bombardement d'électrons de faible puissance, parmi lesquelles on compte la première face 82. De plus, chacune de ces quatre surfaces aura été exposée perpendiculairement à l'axe A du faisceau.

Naturellement, afin d'obtenir un traitement satisfaisant, il est possible de prévoir un asservissement du courant d'alimentation / de la puissance des moyens de stérilisation 18, de sorte qu'à la fin de cette première phase de rotation, chacune des quatre faces présente une unité de dose sensiblement identique et homogène. Cette volonté de faire varier la puissance du faisceau d'électrons durant la rotation de l'objet 2 selon l'axe A' est bien entendu motivée par le fait que ces faces sont exposées au faisceau d'électrons à une distance plus ou moins importante des moyens de stérilisation 18. En effet, à titre d'exemple, la distance verticale entre la base 55 des moyens de stérilisation 18 et la surface extérieure de l'objet 2 traité peut varier entre 85 mm et 215 mm pendant la rotation. Il est noté qu'une alternative pour la régulation de la dose reçue par l'objet pourrait également résider dans le fait d'asservir la position des moyens de stérilisation 18 par rapport à celle de l'objet 2 à traiter.

Après la première phase de rotation, l'objet 2 revient donc dans sa position initiale telle que montrée sur la figure 6a. A cet instant, les quatrièmes moyens de mise en mouvement 67 du second module mécanique 22 sont actionnés de façon à déplacer les moyens de support 64 dans la direction Z, jusqu'à ce que ces derniers viennent au contact de la face inférieure de l'objet 2.

Les pignons motorisés 58 et 60 sont ensuite actionnés à leur tour de manière à ce que les surfaces 38, 40 s'écartent l'une de l'autre, et que leurs picots 49 libèrent le rebord circonférentiel 47 de l'objet 2. Une fois cette opération achevée, l'objet 2 est à nouveau déplacé selon la direction Z par les quatrièmes moyens de mise en mouvement 67, afin que ce même objet soit suffisamment écarté des moyens de préhension 38, 40 et du cadre 52 pour pouvoir être mis en rotation selon l'axe A" sans être gêné.

Effectivement, les troisièmes moyens de mis en mouvement 66 sont actionnés par l'intermédiaire du pignon motorisé 74, dans le but de faire tourner l'objet 2 d'un angle de 90° autour de cet A", ou éventuellement de 270°.

Ensuite, les quatrièmes moyens de mise en mouvement 67 sont à nouveau sollicités pour faire redescendre l'objet 2 jusque dans une position lui permettant de coopérer avec les moyens de préhension 38, 40, eux aussi étant alors à nouveau rapprochés l'un de l'autre de façon à ce que leurs picots 49 engagent respectivement le rebord circonférentiel 47 de deux autres faces de l'objet 2. Lorsque ceci est réalisé, les moyens de support 64 sont déplacés à l'aide du pignon motorisé 76 vers le bas dans leur position d'origine de repos, dans laquelle ils ne présentent aucune gêne pour l'objet 2 en rotation selon l'axe A'.

Comme on peut le voir sur la figure 6b, c'est donc l'une des deux faces orthogonales aux première et seconde faces 82, 80 qui est traversée orthogonalement par l'axe A', cette face dite troisième face étant indiquée par la référence 84 sur la figure 6b. De plus, la première face 82 reste la face supérieure orientée selon un plan XY, et est toujours traversée orthogonalement par l'axe A du faisceau d'électrons.

Les seconds moyens de mise en mouvement 44 du premier module mécanique 12 sont à nouveau actionnés par l'intermédiaire du pignon motorisé 46, afin de mettre l'objet 2 en rotation selon l'axe A'.

Cette seconde phase de rotation de l'objet 2 selon l'axe A', durant laquelle l'axe A du faisceau d'électrons fixe traverse en permanence l'une des faces de l'objet 2, est de préférence opérée jusqu'à ce que cet objet ait effectué un tour complet.

Ainsi, durant cette seconde phase de rotation de l'objet 2 selon l'axe A', ceux sont quatre des six faces de celui-ci qui ont pu être traitées par bombardement d'électrons de faible puissance, parmi lesquelles on compte la seconde face 80 et celle lui étant parallèle et opposée (non référencée), ces deux faces étant les seules à ne pas avoir été traitées lors de la première phase de rotation.

Ici encore, il est noté que pour obtenir un traitement satisfaisant, il est possible de prévoir un asservissement du courant d'alimentation / de la puissance des moyens de stérilisation 18, de sorte qu'à la fin de cette seconde phase de rotation, chacune des quatre faces présente une unité de dose sensiblement identique et homogène. L'alternative selon laquelle on peut opérer un asservissement de la position des moyens de stérilisation 18 par rapport à celle de l'objet 2 à traiter est également envisageable. A titre indicatif, deux des faces ayant déjà été traitées lors de la première phase de rotation de l'objet, l'asservissement prévu pourra être mise en oeuvre de sorte que le traitement de ces deux faces ne constitue qu'un complément minime de traitement par rapport à celui déjà effectué lors de la première phase de rotation. Néanmoins, d'autres solutions sont naturellement envisageables pour assurer la stérilisation des deux faces traversées par l'axe A durant chacune des deux phases de rotation, telles que par exemple celle visant à assurer la moitié du traitement pendant chacune de ces deux rotations, ou encore celle visant à assurer la totalité du traitement requis pendant l'une seulement de ces deux phases.

A la fin de la seconde phase de rotation marquant également la fin de l'illumination de l'objet 2 par les moyens de stérilisation, celui-ci se retrouve dans la position montrée sur la figure 6b, avant d'être déplacé dans la direction X par l'intermédiaire des premiers moyens de mise en mouvement 42. Cette translation est stoppée lorsque l'objet 2 arrive dans le sas de sortie 24, et qu'il se trouve au droit et quasiment au contact du plateau 36 de l'élévateur de sortie 26.

Les pignons motorisés 58 et 60 sont ensuite actionnés de manière à ce que les surfaces 38, 40 s'écartent l'une de l'autre, et que leurs picots 49 libèrent le rebord circonférentiel 47 de l'objet 2. L'objet 2 mis en mouvement par l'élévateur 24 dans une direction opposée à la direction Z, c'est-à-dire vers le bas, est stoppé lorsque le plateau 36 arrive dans une position basse où cet objet 2 est automatiquement transféré par gravité sur le convoyeur de sortie 28 l'amenant jusque dans l'isolateur de production 30.

Il est précisé que le procédé est préférentiellement mis en oeuvre de sorte que pendant la phase de descente de l'objet traité 2 sur le plateau 36, l'objet 2 suivant débute sont parcours au sein de l'installation 1, par exemple en étant déplacé par l'élévateur d'entrée 8 vers le sas d'entrée 10.

Enfin, il est indiqué qu'une stérilisation antérieure de l'installation 1 peut être réalisée en fermant le sas 10 à l'aide du plateau 34 en faisant circuler à travers l'enceinte blindée non étanche un agent stérilisant, provenant de l'isolateur 30, susceptible de s'échapper par un conduit 88 monté à une extrémité libre de la double-paroi protectrice en forme d'enveloppe 49 du premier module mécanique 12, ainsi que par la cheminée 14. Durant cette phase de stérilisation de l'installation 1, il est noté que le plateau 34 est amené dans une position lui permettant d'écraser un joint d'étanchéité (non représenté) prévu autour de l'ouverture du sas d'entrée 10, afin que contrairement à ce qui est recherché pendant la stérilisation des objets 2 par bombardement d'électrons, le sas d'entrée 10 soit fermé de manière étanche. Durant l'opération de stérilisation de l'installation 1, l'agent stérilisant circule alors dans un sens allant de l'isolateur 30 vers le sas d'entrée 10.

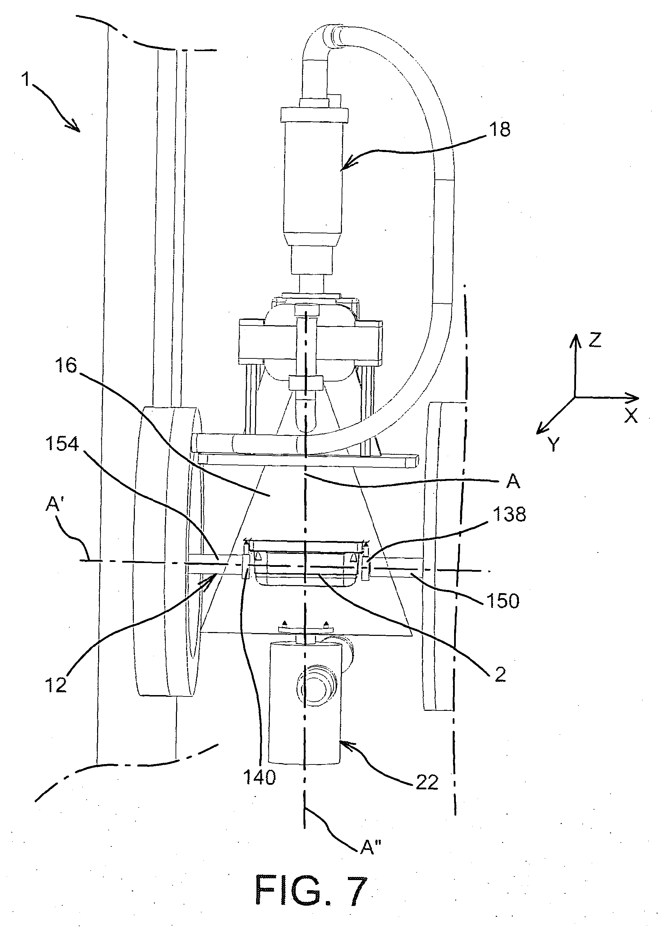

En référence à la figure 7, on peut apercevoir une installation 1 pour la stérilisation d'objets 2 par bombardement d'électrons à faible énergie, cette installation 1 se présentant sous un autre mode de réalisation préféré de la présente invention.

Plusieurs différences sont apparentes par rapport au mode de réalisation représenté sur les figures 1 à 6b, et décrit ci-dessus. Tout d'abord, il est noté qu'une différence majeure réside dans le fait que les moyens de stérilisation 18 sont montés pivotant autour de l'axe A', par l'intermédiaire d'un premier module mécanique (non référencé). En effet, durant le traitement par bombardement d'électrons de faible puissance, ce sont les moyens de stérilisation 18 qui tournent selon l'axe A' autour de l'objet 2 restant fixe.

En outre, cet axe A' reste toujours l'axe selon lequel l'objet 2 est susceptible d'être déplacé en translation à l'intérieur de l'installation 1. Ceci est réalisé à l'aide de deux bras sensiblement coaxiaux 150, 154 portant chacun une surface de préhension 138, 140 destinée à contacter respectivement les deux faces opposées YZ de l'objet 2, comme cela est visible sur la figure 7. Les deux bras 150, 154 sont donc chacun capables de coulisser dans la direction X à l'aide de moyens similaires à ceux présentés dans le mode de réalisation décrit ci-dessus, et chacun d'eux est monté d'un côté de l'installation, par exemple respectivement sur le sas d'entrée 10 et sur le sas de sortie 24.

Par ailleurs, même si cela n'est pas représenté, les élévateurs d'entrée et de sortie peuvent être supprimés, impliquant alors que les convoyeurs d'entrée et de sortie (non représentés sur la figure 7) débouchent directement et respectivement dans les sas d'entrée et de sortie (non représentés sur la figure 7) de l'installation 1. Dans un tel cas, ces convoyeurs forment chicane afin d'assurer la protection biologique.

Il est précisé que chacune des différences exposées ci-dessus peut être intégrée sur l'installation 1 du mode de réalisation représenté sur les figures 1 à 6b, sans sortir du cadre de l'invention.

Bien entendu, diverses modifications peuvent être apportées par l'homme du métier à l'installation 1 et au procédé pour la stérilisation d'objets qui viennent d'être décrits, uniquement à titre d'exemples non limitatifs.

## Revendications

1. Installation (1) pour la stérilisation d'objets par bombardement d'électrons à faible énergie sur la surface extérieure de ces objets, ladite installation comprenant des moyens de stérilisation (18) aptes à générer un faisceau d'électrons à faible énergie selon un axe (A) traversant une chambre de traitement (16) de l'installation, cette installation comprenant en outre un premier module mécanique (12) conçu de manière à pouvoir engendrer, durant la stérilisation d'un objet (2) à traiter situé à l'intérieur de ladite chambre de traitement (16), un mouvement relatif de rotation entre l'objet (2) et lesdits moyens de stérilisation (18), selon un axe (A') choisi de sorte que l'axe (A) dudit faisceau d'électrons généré par lesdits moyens de stérilisation (18) traverse la surface extérieure de l'objet (2) en permanence durant ledit mouvement relatif de rotation selon cet axe (A'), **caractérisée en ce qu'**elle comporte un second module mécanique (22) conçu de manière à pouvoir engendrer, lorsque l'objet (2) situé à l'intérieur de ladite chambre de traitement (16) est libéré du premier module mécanique (12), une mise en rotation dudit objet (2) selon un axe (A") orienté orthogonalement par rapport audit axe (A') et **en ce que** le premier module mécanique (12) est conçu de manière à pouvoir engendrer durant la stérilisation, lorsque l'objet (2) est libéré du deuxième module mécanique (22), ledit mouvement relatif selon l'axe (A').

2. Installation (1) pour la stérilisation d'objets selon la revendication 1, **caractérisée en ce que** ledit premier module mécanique (12) est piloté pour coopérer, durant la stérilisation de l'objet (2) à traiter situé à l'intérieur de ladite chambre de traitement (16), avec l'un quelconque des deux éléments pris parmi ledit objet (2) et lesdits moyens de stérilisation (18), de manière à mettre cet élément en rotation selon ledit axe (A').

3. Installation (1) pour la stérilisation d'objets selon la revendication 1, **caractérisée en ce que** l'objet (2) à traiter constitue ledit élément destiné à coopérer avec le premier module mécanique (12) afin d'être mis en rotation selon ledit axe (A'), et **en ce que** lesdits moyens de stérilisation (18) sont montées fixement sur l'installation.

4. Installation (1) pour la stérilisation d'objets selon la revendication 3, **caractérisée en ce qu'**elle est destinée à la stérilisation d'objets de forme sensiblement parallélépipédique rectangle.

5. Installation (1) pour la stérilisation d'objets selon la revendication 4, **caractérisée en ce que** ledit premier module mécanique (12) est pourvu de moyens de préhension (38, 40) de l'objet (2) à traiter, ledit premier module (12) comportant des premiers moyens de mise en mouvement (42) destinés à amener ledit objet (2), coopérant avec les moyens de préhension (38, 40), dans ladite chambre de traitement (16) de façon à ce qu'une première face (82) de cet objet (2) soit traversée par l'axe (A), le premier module mécanique (12) comprenant également des seconds moyens de mise en mouvement (44) conçus de manière à mettre en rotation ledit objet (2) et les moyens de préhension (38, 40) selon l'axe (A') orienté orthogonalement par rapport à l'axe (A) et par rapport à une seconde face (80) de l'objet (2), et **en ce que** ledit second module mécanique (44) comprend des troisièmes moyens de mise en mouvement (66) conçus de manière à mettre en rotation de 90°, à l'intérieur de ladite chambre de traitement (16), ledit objet (2) libéré desdits moyens de préhension (38, 40), selon l'axe (A") orienté orthogonalement par rapport à l'axe (A') et par rapport à l'une quelconque des faces de l'objet (2) perpendiculaires à ladite seconde face (80).

6. Installation (1) pour la stérilisation d'objets selon la revendication 5, **caractérisée en ce que** ledit second module mécanique (22) comporte des quatrièmes moyens de mise en mouvement (67) conçus de manière à mettre ledit objet (2) en translation selon l'axe (A"), afin d'écarter celui-ci desdits moyens de préhension (38, 40) lors de sa rotation de 90° selon ce même axe (A") engendrée par les troisièmes moyens de mise en mouvement (66).

7. Installation (1) pour la stérilisation d'objets selon la revendication 6, **caractérisée en ce que** ledit premier module mécanique (12) est conçu et piloté de manière à ce que ses moyens de préhension (38, 40) réengagent l'objet (2) de forme sensiblement parallélépipédique rectangle ayant subi la rotation de 90° générée par les troisièmes moyens de mise en mouvement (66), afin que cet objet (2) puisse être mis en rotation selon l'axe (A') par les seconds moyens de mise en mouvement (44).

8. Installation (1) pour la stérilisation d'objets selon la revendication 7, **caractérisée en ce que** ledit axe (A") est orienté selon une direction de la hauteur (Z) de l'installation, et **en ce que** ledit axe (A) est orienté selon une direction d'avancement des objets (X) au sein de l'installation, les directions (X) et (Z) étant orthogonales entre elles.

9. Installation (1) pour la stérilisation d'objets selon la revendication 8, **caractérisée en ce que** ledit axe (A) est également orienté selon la direction de la hauteur (Z) de l'installation.

10. Installation (1) pour la stérilisation d'objets selon la revendication 8 ou la revendication 9, **caractérisée en ce qu'**elle comprend en outre un sas d'entrée (10) et un sas de sortie (24) entre lesquels se situe ladite chambre de traitement (16) communiquant avec celles-ci, cette chambre de traitement (16) ainsi que les deux sas d'entrée et de sortie (10, 24) étant alignés selon ladite direction (X).

11. Installation (1) pour la stérilisation d'objets selon la revendication 10, **caractérisée en ce que** lesdits premier moyens de mise en mouvement (42) sont conçus de manière à permettre un déplacement en translation selon la direction (X) de l'objet (2) maintenu par les moyens de préhension (38, 40), entre le sas d'entrée (10) et la chambre de traitement (16), ainsi qu'entre cette dernière et le sas de sortie (24).

12. Installation (1) pour la stérilisation d'objets selon la revendication 10 ou la revendication 11, **caractérisée en ce que** les deux sas d'entrée et de sortie (10, 24) en position fermée, et ladite chambre de traitement (16), forment conjointement une enceinte blindée non étanche.

13. Installation (1) pour la stérilisation d'objets selon l'une quelconque des revendications 10 à 12, **caractérisée en ce que** chacun des deux sas d'entrée et de sortie (10, 24) est associé à un élévateur (8, 26) orienté selon la direction (Z).

14. Installation (1) pour la stérilisation d'objets selon la revendication 13, **caractérisée en ce que** chacun des deux élévateurs (8, 26) comporte un plateau mobile blindé (34, 36) servant de support pour l'objet (2), ledit plateau (34, 36) étant conçu pour assurer la fermeture du sas (10, 24) auquel il est associé.

15. Installation (1) pour la stérilisation d'objets selon la revendication 13 ou la revendication 14, **caractérisée en ce que** les deux élévateurs (8, 26) coopèrent respectivement avec un convoyeur d'entrée (6) et un convoyeur de sortie (28), chacun apte à assurer le déplacement desdits objets.

16. Installation (1) pour la stérilisation d'objets selon la revendication 15, **caractérisée en ce que** ledit convoyeur de sortie (28) débouche dans un isolateur de production (30) dans lequel sont acheminés les objets stérilisés.

17. Installation (1) pour la stérilisation d'objets selon l'une quelconque des revendications 10 à 16, **caractérisée en ce que** ledit premier module mécanique (12) est monté fixement sur ledit sas d'entrée (10).

18. Installation (1) pour la stérilisation d'objets selon l'une quelconque des revendications précédentes, **caractérisée** les moyens de stérilisation (18) aptes à générer un faisceau d'électrons à faible énergie prennent la forme d'un canon à électrons monté à la périphérie de ladite chambre de traitement (16).

19. Procédé de stérilisation d'objets par bombardement d'électrons à faible énergie sur la surface extérieure de ces objets, **caractérisé en ce qu'**il est mis en oeuvre à l'aide d'une installation (1) selon l'une quelconque des revendications précédentes.

## Claims

1. Installation (1) for sterilising objects by low-energy electron bombardment on the external surface of said objects, which installation includes sterilisation means (18) capable of generating a low-energy electron beam along an axis (A) passing through a treatment chamber (16) of the installation, which installation also includes a first mechanical module (12) designed so as to be capable of generating, during the sterilisation of an object (2) to be treated located inside the treatment chamber (16), a relative rotation movement between the object (2) and said sterilisation means (18), about an axis (A') chosen so that axis (A) of said electron beam generated by said sterilisation means (18) continuously passes through the external surface of the object (2) during the relative rotation movement about said axis (A'), **characterised in that** it comprises a second mechanical module (22) designed so as to be capable of generating, when the object (2) located inside said treatment chamber (16) is released from the first mechanical module (12), a rotation of the object (2) about an axis (A") oriented orthogonally with respect to axis (A') and **in that** the first mechanical module (12) is designed so as to be capable of generating, during sterilisation, when the object (2) is released from the second mechanical module (22), said relative movement according to the axis (A').

2. Installation (1) for sterilising objects according to claim 1, **characterised in that** said first mechanical module (12) is controlled so as to cooperate, during the sterilisation of the object (2) to be treated located inside said treatment chamber (16), with any one of the two elements selected from said object (2) and said sterilisation means (18) so as to cause said element to rotate about said axis (A').

3. Installation (1) for sterilising objects according to claim 1, **characterised in that** the object (2) to be treated constitutes said element intended to cooperate with the first mechanical module (12) so as to be moved in rotation about said axis (A'), and **in that** said sterilisation means (18) are mounted stationarily on the installation.

4. Installation (1) for sterilising objects according to claim 3, **characterised in that** it is intended for sterilising objects having a substantially rectangular parallelepiped shape.

5. Installation (1) for sterilising objects according to claim 4, **characterised in that** said first mechanical module (12) is provided with means (38, 40) for gripping the object (2) to be treated, which first module (12) comprises first movement means (42) intended to bring said object (2), cooperating with the gripping means (38, 40), into the treatment chamber (16) so that a first surface (82) of this object (2) is passed through by the axis (A), wherein the first mechanical module (12) also includes second movement means (44) designed so as to rotate the object (2) and the gripping means (38, 40) about the axis (A') oriented orthogonally with respect to the axis (A) and with respect to a second surface (80) of the object (2), and **in that** the second mechanical module (44) includes third movement means (66) designed so as to rotate the object (2) released from the gripping means (38, 40) at 90°, inside the treatment chamber (16), about the axis (A") oriented orthogonally with respect to the axis (A') and with respect to any one of the surfaces of the object (2) which are perpendicular to the aforementioned second surface (80).

6. Installation (1) for sterilising objects according to claim 5, **characterised in that** said second mechanical module (22) comprises fourth movement means (67) designed so as to move said object (2) in translation according to the axis (A"), so as to distance it from said gripping means (38, 40) during its rotation at 90° about said same axis (A") caused by the third movement means (66).

7. Installation (1) for sterilising objects according to claim 6, **characterised in that** said first mechanical module (12) is designed and controlled so that its gripping means (38, 40) re-engage the object (2) having a substantially rectangular parallelepiped shape which has been moved in rotation at 90° by the third movement means (66), so that said object (2) can be moved in rotation about the axis (A') by the second movement means (44).

8. Installation (1) for sterilising objects according to claim 7, **characterised in that** said axis (A") is oriented in the direction of the height (Z) of the installation, and **in that** said axis (A) is oriented in the direction of forward movement of the objects (X) in the installation, which directions (X) and (Z) are mutually orthogonal.

9. Installation (1) for sterilising objects according to claim 8, **characterised in that** said axis (A) is also oriented in the direction of the height (Z) of the installation.

10. Installation (1) for sterilising objects according to claim 8 or claim 9, **characterised in that** it also includes an inlet lock (10) and an outlet lock (24) containing therebetween said treatment chamber (16) communicating with said locks (10, 24), which treatment chamber (16) as well as the two inlet and outlet locks (10, 24) are aligned in said direction (X).

11. Installation (1) for sterilising objects according to claim 10, **characterised in that** said first movement means (42) are designed so as to allow for a translation movement in direction (X) of the object (2) held by the gripping means (38, 40), between the inlet lock (10) and the treatment chamber (16), as well as between the latter and the outlet lock (24).

12. Installation (1) for sterilising objects according to claim 10 or claim 11, **characterised in that** the two inlet and outlet locks (10, 24) in closed position, and said treatment chamber (16), jointly form an unsealed, shielded enclosure.

13. Installation (1) for sterilising objects according to any one of claims 10 to 12, **characterised in that** each of the two inlet and outlet locks (10, 24) is associated with an elevator (8, 26) oriented in the direction (Z).

14. Installation (1) for sterilising objects according to claim 13, **characterised in that** each of the two elevators (8, 26) comprises a shielded mobile plate (34, 36) serving as a carrier for the object (2), which plate (34, 36) is designed so as to ensure the closure of the lock (10, 24) with which it is associated.

15. Installation (1) for sterilising objects according to claim 13 or claim 14, **characterised in that** the two elevators (8, 26) cooperate respectively with an inlet conveyor (6) and an outlet conveyor (28), each capable of moving said objects.

16. Installation (1) for sterilising objects according to claim 15, **characterised in that** said outlet conveyor (28) leads to a production isolator (30) into which the sterilised objects are delivered.

17. Installation (1) for sterilising objects according to any one of claims 10 to 16, **characterised in that** said first mechanical module (12) is mounted stationarily on said inlet lock (10).

18. Installation (1) for sterilising objects according to any one of the previous claims, **characterised in that** the sterilisation means (18) capable of generating a low-energy electron beam take the form of an electron gun mounted at the periphery of said treatment chamber (16).

19. Method for sterilising objects by low-energy electron bombardment on the external surface of said objects, **characterised in that** it is implemented using an installation (1) according to any one of the previous claims.

## Patentansprüche

1. Anlage (1) zum Sterilisieren von Objekten durch Beschuß von Elektronen niedriger Energie auf die äußere Oberfläche dieser Objekte, wobei die Anlage Sterilisationsmittel (18) umfaßt, die dazu ausgelegt sind, einen Elektronenstrahl niedriger Energie entlang einer Achse (A) zu erzeugen, die eine Behandlungskammer (16) der Anlage durchsetzt, wobei diese Anlage ferner ein erstes mechanisches Modul (12) umfaßt, das dazu ausgelegt ist, dass es während des Sterilisierens eines zu behandelnden Objekts (2), das sich im Inneren der Behandlungskammer (16) befindet, eine relative Drehbewegung zwischen dem Objekt (2) und den Sterilisationsmitteln (18) um eine Achse (A') erzeugen kann, die derart gewählt ist, dass die Achse (A) des Elektronenstrahls, der durch die Sterilisationsmittel (18) erzeugt wird, die äußere Oberfläche des Objekts (2) ständig während der relativen Drehbewegung um diese Achse (A') durchsetzt, **dadurch gekennzeichnet, dass** sie ein zweites mechanisches Modul (22) umfaßt, das dazu ausgelegt ist, dass es, während das Objekt (2), das sich im Inneren der Behandlungskammer (16) befindet, vom ersten mechanischen Modul (2) befreit wird, ein Indrehungversetzen des Objekts (2) um eine Achse (A") erzeugen kann, die orthogonal zur Achse (A') orientiert ist, und dass das erste mechanische Modul (12) dazu ausgelegt ist, dass es während der Sterilisation, wenn das Objekt (2) vom zweiten mechanischen Modul (22) befreit ist, die Relativbewegung um die Achse (A') erzeugen kann.

2. Anlage (1) zum Sterilisieren von Objekten nach Anspruch 1, **dadurch gekennzeichnet, dass** das erste mechanische Modul (12) dazu gesteuert wird, während der Sterilisation des zu behandelnden Objekts (2), das sich im Inneren der Behandlungskammer (16) befindet, mit einem beliebigen der beiden Elemente zusammenzuwirken ausgewählt aus dem Objekt (2) und den Sterilisationsmitteln (18), derart, dass dieses Element in Drehung um die Achse (A') versetzt wird.

3. Anlage (1) zum Sterilisieren von Objekten nach Anspruch 1, **dadurch gekennzeichnet, dass** das zu behandelnde Objekt (2) das Element bildet, das dazu bestimmt ist, mit dem ersten mechanischen Modul (12) zusammenzuwirken, um in Drehung um die Achse (A') versetzt zu werden, und dass die Sterilisationsmittel (18) fest an der Anlage montiert sind.

4. Anlage (1) zum Sterilisieren von Objekten nach Anspruch 3, **dadurch gekennzeichnet, dass** sie zum Sterilisieren von Objekten von im wesentlichen rechtwinklig-parallelepipedförmiger Gestalt ausgelegt ist.

5. Anlage (1) zum Sterilisieren von Objekten nach Anspruch 4, **dadurch gekennzeichnet, dass** das erste mechanische Modul (12) mit Mitteln (38, 40) zum Ergreifen des zu behandelnden Objekts (2) ausgestattet ist, wobei das erste Modul (12) erste Inbewegungversetzmittel (42) umfaßt, die dazu ausgelegt sind, das Objekt (2) kooperierend mit den Greifmitteln (38, 40) in die Behandlungskammer (16) derart zu bringen, dass eine erste Seite (82) dieses Objekts (2) von der Achse (A) durchsetzt wird, wobei das erste mechanische Modul (12) ferner zweite Inbewegungversetzmittel (44) umfaßt, die dazu ausgelegt sind, das Objekt (2) und die Greifmittel (38, 40) in Drehung um die Achse (A') zu versetzen, die orthogonal zur Achse (A) und zu einer zweiten Seite (80) des Objekts (2) orientiert ist, und dass das zweite mechanische Modul (44) dritte Inbewegungversetzmittel (66) umfaßt, die dazu ausgelegt sind, das von den Greifmitteln (38, 40) befreite Objekt (2) im Inneren der Behandlungskammer (16) in Drehung um 90 Grad um die Achse (A") zu versetzen, die orthogonal zur Achse (A') und zu einer beliebigen der Seiten des Objekts (2) ist, die senkrecht zu der zweiten Seite (80) sind.

6. Anlage (1) zum Sterilisieren von Objekten nach Anspruch 5, **dadurch gekennzeichnet, dass** das zweite mechanische Modul (22) vierte Inbewegungversetzmittel (67) umfaßt, die dazu ausgelegt sind, das Objekt (2) in Translation entlang der Achse (A") zu versetzen, um es von den Greifmitteln (38, 40) während seiner Drehung um 90 Grad um diese selbe Achse (A") zu entfernen, die von den dritten Inbewegungversetzmitteln (66) erzeugt wird.

7. Anlage (1) zum Sterilisieren von Objekten nach Anspruch 6, **dadurch gekennzeichnet, dass** das erste mechanische Modul (12) dazu ausgelegt und gesteuert ist, dass seine Greifmittel (38, 40) wieder an dem Objekt (2) mit im wesentlichen rechteckig-parallelepipedförmiger Gestalt angreifen, das die Drehung um 90 Grad durchlaufen hat, welche von den dritten Inbewegungversetzmitteln (66) erzeugt wurde, damit dieses Objekt (2) durch die zweiten Inbewegungversetzmittel (44) in Drehung um die Achse (A') versetzt werden kann.

8. Anlage (1) zum Sterilisieren von Objekten nach Anspruch 7, **dadurch gekennzeichnet, dass** die Achse (A") entlang einer Höhenrichtung (Z) der Anlage orientiert ist, und dass die Achse (A) entlang einer Vorrückrichtung der Objekte (X) im Inneren der Anlage orientiert ist, wobei die Richtungen (X) und (Y) orthogonal zueinander sind.

9. Anlage (1) zum Sterilisieren von Objekten nach Anspruch 8, **dadurch gekennzeichnet, dass** die Achse (A) ebenfalls entlang der Höhenrichtung (Z) der Anlage orientiert ist.

10. Anlage (1) zum Sterilisieren von Objekten nach Anspruch 8 oder nach Anspruch 9, **dadurch gekennzeichnet, dass** sie ferner eine Eingangsschleuse (10) und eine Ausgangsschleuse (24) umfaßt, zwischen denen sich die Behandlungskammer (16) befindet, die mit diesen in Verbindung steht, wobei diese Behandlungskammer (16) ebenso wie die Eingangs- und die Ausgangsschleuse (10, 24) entlang der Richtung (X) ausgerichet sind.

11. Anlage (1) zum Sterilisieren von Objekten nach Anspruch 10, **dadurch gekennzeichnet, dass** die ersten Inbewegungversetzmittel (42) dazu ausgelegt sind, eine Translationsverlagerung des Objekts (2), das von den Greifmitteln (38, 40) gehalten wird, entlang der Richtung (X) zwischen der Eingangsschleuse (10) und der Behandlungskammer (16) sowie zwischen dieser letztgenannten und der Ausgangsschleuse (24) zu ermöglichen.

12. Anlage (1) zum Sterilisieren von Objekten nach Anspruch 10 oder nach Anspruch 11, **dadurch gekennzeichnet, dass** die Eingangs- und die Ausgangsschleuse (10, 24) in geschlossener Position sowie die Behandlungskammer (16) gemeinsam einen nicht-dichten abgeschirmten Behälter bilden.

13. Anlage (1) zum Sterilisieren von Objekten nach einem der Ansprüche 10 bis 12, **dadurch gekennzeichnet, dass** jede einzelne von der Eingangs- und der Ausgangsschleuse (10, 24) einer Hebevorrichtung (8, 26) zugeordnet ist, die entlang der Richtung (Z) orientiert ist.

14. Anlage (1) zum Sterilisieren von Objekten nach Anspruch 13, **dadurch gekennzeichnet, dass** jede der zwei Hebevorrichtungen (8, 26) eine bewegliche abgeschirmte Platte (34, 36) umfaßt, die als Unterstützung für das Objekt (2) dient, wobei die Platte (34, 36) dazu ausgelegt ist, das Schließen der Schleuse (10, 24) sicherzustellen, der sie zugeordnet ist.

15. Anlage (1) zum Sterilisieren von Objekten nach Anspruch 13 oder nach Anspruch 14, **dadurch gekennzeichnet, dass** die zwei Hebevorrichtungen (8, 26) mit einem Eingangsförderer (6) bzw. mit einem Ausgangsförderer (28) zusammenwirken, von denen jeder dazu ausgelegt ist, die Verlagerung der Objekte sicherzustellen.

16. Anlage (1) zum Sterilisieren von Objekten nach Anspruch 15, **dadurch gekennzeichnet, dass** der Ausgangsförderer (28) in einen Produktionsisolator (30) mündet, in den die sterilisierten Objekte geleitet werden.

17. Anlage (1) zum Sterilisieren von Objekten nach einem der Ansprüche 10 bis 16, **dadurch gekennzeichnet, dass** das erste mechanische Modul (12) fest an der Eingangsschleuse (10) montiert ist.

18. Anlage (1) zum Sterilisieren von Objekten nach einem der vorhergehenden Ansprüche, **dadurch gekennzeichnet, dass** die Sterilisationsmittel (18), die dazu ausgelegt sind, einen Elektronenstrahl niedriger Energie zu erzeugen, die Gestalt einer Elektronenkanone aufweisen, die an der Peripherie der Behandlungskammer (16) montiert ist.

19. Verfahren zum Sterilisieren von Objekten durch Beschuß von Elektronen niedriger Energie auf die äußere Oberfläche dieser Objekte, **dadurch gekennzeichnet, dass** es mit Hilfe einer Anlage (1) nach einem der vorhergehenden Ansprüche durchgeführt wird.
